# EUROPEAN PATENT SPECIFICATION

(11) **EP 1 722 697 B1**
(45) Date of publication and mention of the grant of the patent: **24.11.2010**
(21) Application number: 05724826.2
(22) Date of filing: 07.03.2005
(51) Int. Cl.: A61B 17/32, A61B 17/22

(54) **SMALL-DIAMETER SNARE**
SCHLINGE MIT KLEINEM DURCHMESSER
ANSE DE PETIT DIAMETRE

(30) Priority: 08.03.2004 US 551313 P
(43) Date of publication of application: 22.11.2006
(73) Proprietor: Radius Medical Technologies, Inc., Acton, MA 01720 (US)
(72) Inventor: DEMELLO, Richard, M., Stow, MA 01775 (US); FINLAYSON, Maureen, A., Stow, MA 01775 (US); FLIGHT, Bruce, W., Melrose, MA 02176 (US); BURKHARDT, Jon, Ashland, MA 01721 (US)
(74) Representative: Rupprecht, Kay
(86) International application number: PCT/US2005/007361
(87) International publication number: WO 2005/087119

(56) References cited:
- WO-A-94/06357
- WO-A-2004/006789
- US-A- 5 234 425
- US-A- 5 906 622
- US-A1- 2001 031 970

## Description

### BACKGROUND OF THE INVENTION

### Field of the Invention

The present invention relates to surgical snares, and more particularly to devices for retrieving broken, dislodged, or separated medical devices from within the vascular system.

### Background Information

Certain snare devices have become available over recent years for retrieving malfunctioning or misplaced devices within the cardiovascular and non-vascular regions of the body. These typically consist of fairly large diameter sheaths, which house a movable central wire or wires whose distal ends are formed into a loop or loops. The loop is used to ensnare and capture the desired object for withdrawal and removal from the body. In use, the snare is typically passed through a guiding catheter or other introducing catheter that is placed within the vasculature and is directed to the vessel or area where the misplaced or malfunctioning device is located. The snare can then capture the intended device and retrieve it out of the body through the introducing catheter or by withdrawing both the snare and the introducing catheter in tandem.

Currently available snares are designed using large diameter outer sheaths that require larger entry sites. This may result in complications such as excessive bleeding and/or hematomas. Additionally, because of the large diameter, it may be necessary to remove the existing catheters and exchange to other larger devices increasing the overall time and cost of the procedure. A third disadvantage of the old means is that the outer sheath, which is typically made of a plastic material, exhibits little or no torque control, which can make ensnaring the misplaced or malfunctioned device very difficult. Lastly, because of the size and stiff design of these snare devices, they have a very sharp distal leading edge which cannot be safely advanced into small diameter vessels such as those in the coronary and cerebral vasculature without risking damage to the vessel wall. An exemplary small-diameter snare design that satisfies many of the concerns above is provided in commonly owned U.S. Patent No. 6,554,842, entitled SMALL DIAMETER SNARE by Heuser, *et al.*

Devices, such as the exemplary Heuser design, are characterized by a small-diameter outer sheath that has a relatively thin wall (for example, approximately 50.8 µm (0.0020 inch) or less in wall thickness) so as to accommodate an axially movable/rotatable central core wire of approximately 203.2 µm (0.008 inch). The structure allows a snare loop attached to the distal end of the core wire and housed within the open distal end of the sheath to be selectively extended from the sheath end, withdrawn and torqued. This sheath is at least partially composed of metal. The thinness of the tube, and it's metallic content make it susceptible to splitting, fracturing and fatigue failure under stress. In addition, the metal section of the tubular outer sheath tends to experience permanent (plastic) deformation when bent, and once deformed, the central core wire will tend to bind upon the lumen of the sheath, rendering the device inoperable for its intended purpose. In addition, the outer wall of the metal tube section has a lubricious coating, such as PTFE (Teflon), which is typically approximately 25.4 µm (0.0010 inch) in thickness. This necessitates further downsizing of the sheath overall outer diameter thereby reducing the inner diameter available for accommodating the central core wire, thereby further increasing the risk of inadvertent failure of the device through breakage or plastic deformation.

### SUMMARY OF THE INVENTION

This invention overcomes prior disadvantages by providing a small-diameter snare device according to claim 1, with preferred embodiments in the dependent claims.

### BRIEF DESCRIPTION OF THE DRAWINGS

The invention description below refers to the accompanying drawings, of which:
Fig. 1 is a partial side cross section of a small-diameter snare device according to an illustrative embodiment of this invention;
Fig. 2 is a full cross section in the region of the attachment between the loop and core wire, taken along line 2-2 of Fig.1;
Fig. 3 is a cross section of a snare loop wire according to an alternate embodiment having a braided construction;
Fig. 4 is a partial side cross section of the small-diameter snare device including a manipulator handle assembly attached to the proximal end thereof;
Fig. 5 is a full cross section in the region of the slide actuator of the handle, taken along line 5-5 of Fig. 4; and
Fig. 6 is a somewhat cross section of a pair of D-shaped loop wire sections adjacent to the region of their connection to the core wire according to an alternate embodiment.

### DETAILED DESCRIPTION OF AN ILLUSTRATIVE

### EMBODIMENT

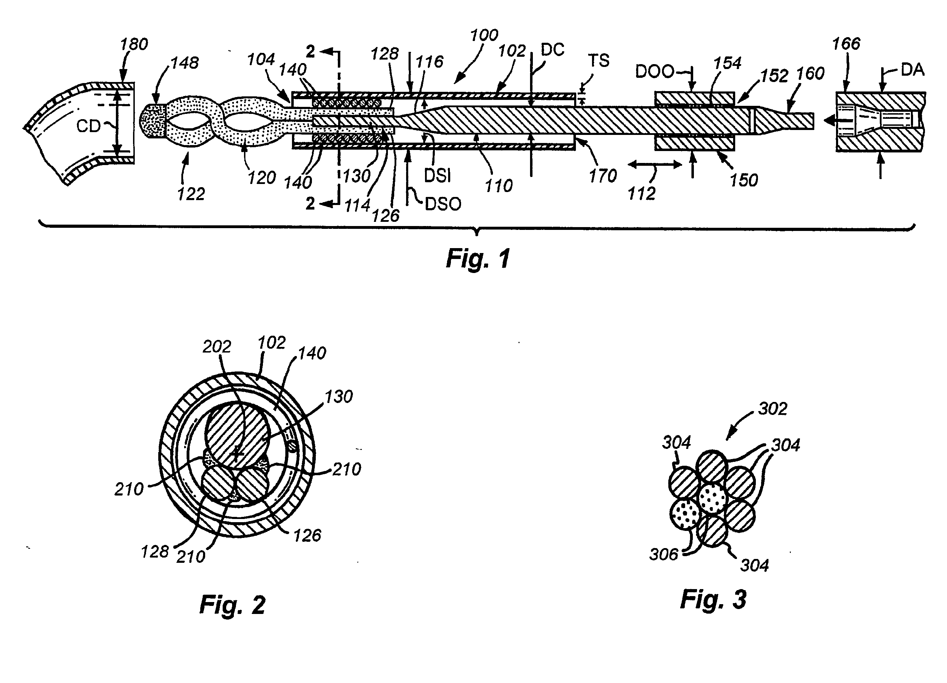

Fig. 1 shows a small diameter snare device 100 according to an embodiment of this invention. The device 100 includes of a hollow, elongate, thin-walled polymer outer sheath 102. The sheath 102 may include a radiopaque marker located at or adjacent to the open distal end 104 for visualization under fluoroscopy. The polymer can be any one of a number of acceptable biocompatible polymers with sufficient structural strength to support a thin-walled (approximately 50.8 µm (0.0020 inch) maximum wall thickness TS) structure without rupture or other failure under normal use conditions.

In one embodiment, the sheath is constructed from polyimide with a tungsten filler for radiopacity. The radiopaque filler may be added to the sheath polymer during processing, or a radiopaque material may be added to the outer surface via vapor deposition, plating, ion implantation processes, or the like. Alternatively, radiopaque markers can be applied at the distal end and/or other known locations along the sheath, and thus, an overall tungsten filler/radiopaque coating can be omitted. As discussed further below, the outer surface can include thereon a polytetrafluoroethylene (PTFE or "Teflon") coating upon some, or all, of its outer surface for enhanced lubricity. Alternatively, the outer sheath coating can be constructed form a hydrophilic material that provides lubricity, instead of a PTFE coating. The sheath polyimide material is commercially available for a variety of vendors and sources and is becoming accepted in a variety of medical device applications. It has the property of allowing a very strong, thin-walled cylindrical-cross section tube to be made therefrom, with wall thicknesses on the order of approximately 19.05 µm (0.00075 inch) to 254 µm (0.010 inch) in normal applications Nevertheless, the resulting polyimide tube can withstand high pressures in excess of 5.171 x 10⁶ Pa (750 psi) when employed in the size range of the sheath of this invention. Polyimide also resists high temperatures, as much as 537.8°C (1000 degrees F), or greater. Accordingly, polyimide is desirable as a sheath material based upon all of the above-described superior performance characteristics. Nevertheless, it is expressly contemplated that other equivalent plastic/polymer materials suitable for forming a thin-walled sheath tube with similar or better properties (e.g. high strength, thin wall-thickness limits, small diametric sizing) may also be employed as an acceptable "polymer" herein.

The outer sheath 102, which forms the main support and outer framework of the device 100 has an overall length sufficient to traverse the body's varied vasculature, and is (for most applications) permissibly in a range of between approximately 20 cm and 500 cm (more typically between 120 cm and 300 cm). The outer diameter DSO of the sheath is permissibly (for most applications) in a range of between approximately 254 µm (0.010 inch) to 1.143 mm (0.045 inch) not according to the invention, (more typically between 254 µm (0.010 inch) and 533 µm (0.021 inch) according to the invention. In general, where the outer diameter is less than 8.89 mm (0.35 inch), the device 100 may fit easily through a standard balloon catheter.

A single central core wire 110 extends through the entire length of the sheath 102. The outer diameter DC of the core wire through most of the length of the sheath 102 (except near the distal end 104) is sized close to the inner diameter DSI of the sheath while allowing for axial sliding (double arrow 112), in order to maximize the support imparted by the core wire 110 to the body portion/sheath 102 of the snare device 100. The distal end 114 of the core wire 110 has a tapered section 116 of reduced diameter or cross section to provide a "guidewire-like" flexibility to the distal portion of the device. A second (typically metal) wire 120 of about 50% the inner diameter of the sheath is shaped to form a snare loop 122, and the two ends 126 and 128 are attached to the distal-most portion 130 of the central core wire 110 via welding, soldering, brazing or another high-strength (typical metal-flowing). The loop 122 is typically circular or oval shaped and can also be multiplanar (a twisted "figure-eight" as shown, for example) so as to increase the ability to ensnare and capture objects. Where a multi-planar structure is shown, the entire structure can be referred to collectively as a loop or the two resulting oval perimeters in the figure-eight can be termed in the plural as "loops." The loop or loops can have a permissible diametric range (their object-grasping inner circumference) of between approximately 1mm and 100mm, and typically have a range between 2mm and 35mm. However ranges outside the stated values are expressly contemplated.

The central core wire 110 is made from metal for flexibility and strength. In one embodiment, the central core wire 110 may be made by connecting a proximal stainless steel portion, for support and stiffness, to a distal nitinol portion, for torque-ability and kink resistance. Likewise, it can be made from 300 series stainless steel or a stronger, heat settable material such as 400 series stainless steel, alloy MP35N, a chromium-cobalt alloy such as Elgiloy, or nitinol in its super elastic or linear elastic state.

Note, because a thin-walled polymer sheath is employed, it advantageously allows for a maximized central core wire diameter, which in turn, provides stiffness for torque control and axial pushability in the body of the snare device.

With reference also to Fig. 2, the central core wire distal-most portion 130 may be offset in one axis relative to the central axis 202 of the sheath 102. This allows mounting of the loop ends 126 and 128 in a most efficient cross-sectional-space-saving manner. Surrounding the distal most portion and two loop ends 126, 128 is a helical wrap of platinum (in this embodiment) wire 140. In one embodiment, the wire has a diameter of approximately 25.4 µm (0.001 inch). It is applied to the interconnection between the loop and core wire prior to permanent joining-together of the structure. It thereby secures these components in a tight relationship while solder, weldment, brazing, etc. are applied, reducing the risk of unwanted separation/spreading or sliding of components relative to each other. This further ensures a predictable end diameter for the structure, allowing a tighter wall-thickness tolerance without risk of binding between the core wire assembly and inner wall of the sheath 102. During assembly, solder, etc. passes through small gaps formed between wraps of wire 140 to pool in fillets 210 against the components 126, 128 and 130.

In one embodiment, the snare loop 122 may be made from a 300 series or a heat settable material, such as 400 series stainless steel material, MP35N. Likewise, it may be made from a kink-resistant material, such as chromium-cobalt or nitinol alloy. The snare loop may have an optional radiopaque marker 148 located at the distal-most portion of the loop 122 to aid in fluoroscopic visualization. Alternatively, the snare loop(s) may be formed of a radiopaque material, such as platinum to aid in fluoroscopic visualization. Similarly, the snare loops may have a radiopaque coating applied via vapor deposition, plating, ion implantation processes, or the like, to aid in fluoroscopic visualization, or the snare loop(s) may be covered by a coil (not shown) wound from a radiopaque material, such as platinum to aid in fluoroscopic visualization.

In another embodiment (see Fig. 3), the snare loop(s) may be formed of a wire 302 that defines a plurality of stranded or braided members 304 of an appropriate wire strand material, rather than a single, solid wire as shown above. This stranded or braided wire 302 may (in one or more embodiments) include at least one strand (or multiple strands) 306 of a radiopaque material, such as platinum to aid in fluoroscopic visualization. Alternatively, the snare loop(s) may be formed of a radiopaque material-cored tube, such as a tantalum filled chromium-cobalt material, or platinum filled nitinol material (not shown).

While the snare loops are shown as an independent component attached to a separate core wire end, it is expressly contemplated that the core wire and loops can be a unitary component. For example, in an alternate embodiment (not shown), the snare loops can be made from part of the central core by reducing the diameter of the end of the central core and doubling this free distal end over to form the loop. The free distal end is then joined to the more-proximal part of the narrowed distal end of the core wire. The joint can include wrapping with wire (130 above) and soldering, etc. to construct the finished loop structure.

After assembly of the core wire 110 with appropriate loop(s), and its insertion into the sheath 102, a second short, hollow tube is fitted over the proximal end 152 of the central core wire 110 and attached thereto by a filler or adhesive 154 to provide an actuating handle 150 so as to slideably move the central core wire axially (double arrow 112) within the sheath 102, thus selectively exposing and retracting the snare loop 122 from the open distal end 104 of the sheath 102. In one embodiment, the actuating handle 150 may be sized with an outer diameter DOO similarly (or identically) in outer diameter DSO to the main body of the sheath 102. The exposed proximal end 152 of the core wire 110 may include a narrowed-diameter end 160, with a special connection so that an additional length of wire 166 can be attached to it, thereby extending the overall length of the snare device. This extension has a similarly sized outer diameter DA to that of the handle 150 (DOO) and sheath 102 (DSO). The attachment of this similarly small-diameter extension allows for the exchange of one catheter for another catheter over the body of the snare (and extension). The entire snare device when complete (including the actuating handle 150) can be made less than 355.6 µm (.014 inch) in overall outer diameter, and is therefore capable of being placed directly through a PTCA balloon catheter or other small-diameter catheter 180 (Fig. 1), having a sufficiently large inner diameter CD, that may already be in place within the patient (e.g. CD > DSO). Since the actuating handle is equally small in diameter, it also passes through the small-diameter catheter with an extension piece joined behind the handle to the attachment end 160, and thereby allowing the device to be guided even deeper into the patient when needed. The snare may also be passed through the guiding catheter along side of the balloon or access catheter without the need to remove the prior device and, thus, lose temporary access to the site within the patient. For example, the snare may be initially passed through the PTCA balloon catheter, which is already located within the target area. The balloon catheter can then be removed and replaced with a larger-inner diameter catheter to allow removal of the object.

The actuating handle 150 may consist of a metal or a polymer tube. In an alternate embodiment (not shown) the actuating handle may consist of a tube slideable within a second metal tube that is attached to the proximal end 170 of the sheath to maintain an axial orientation between the proximal end of the core wire 102 and sheath, thereby minimizing permanent bending or kinking of the core wire at or near this proximal location.

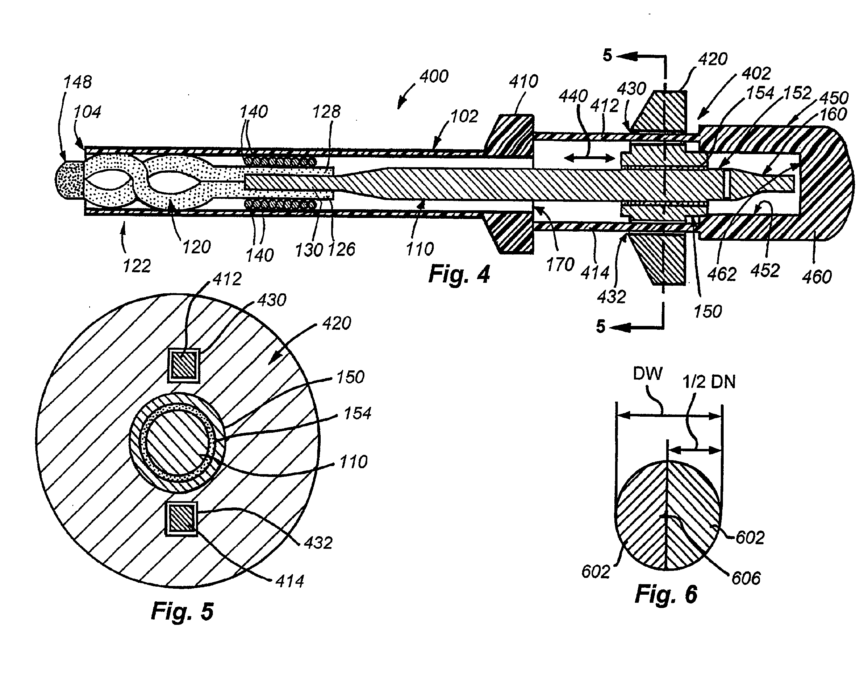

While the depicted actuating handle 150 is of similar outer diameter as the sheath 102, it is expressly contemplated (where the handle will not be passed into another catheter) that the actuating handle may be made in a diameter significantly larger than the snare device so that it may also serve as a torquing handle, similar to those utilized in routine small-diameter guidewire placement. Fig. 4 shows an overall version 400 of the snare device that includes an enlarged handle attachment 402 attachment to the previously described snare device of Fig. 1 (with like components in Figs. 1 and 4 retaining like reference numbers). The handle attachment 402 may be made from a polymer material which (in an embodiment of this invention) is injection molded and attached onto the snare or (in another embodiment) may be over molded directly onto the snare. The handle attachment 402 includes a base ring 410 that is secured to the outer surface of the proximal end 170 of the sheath 102. In a detachable-handle embodiment, the ring can consist of a conventional lockable collet structure in which turning of an outer element reduces the diameter of an inner locking element to deliver securing hoop stress to the distal end 170 outer surface of the sheath 102. The base ring is connected to two or more ribs 412 and 414 that are also shown in cross section in Fig. 5.

An actuating ring 420 is secured onto the actuating handle 150 either permanently or detachably. Where it is detachable, it may also utilize a locking collet structure (not shown) as described above. At least two apertures 430 and 432 allow passage of the respective ribs 412 and 414 so that the ring 420, actuating handle 150 and core wire 110 can be slid axially (double arrow 440) with respect to the sheath 102 based upon slideable movement of the actuating ring 420. The ribs secure the ring 420 and interconnected core wire 110 and handle 150 against rotation relative to the sheath. The connection is sufficiently strong so that rotation of the handle assembly 402 causes torquing of the entire device so as to rotate the loop(s) 122 into a desired rotational orientation. In an alternate embodiment, the ring may be a non-circular structure. In another alternate embodiment (also not shown), the ring 420 may also allow at least limited rotation of the core wire relative to the sheath by utilizing arcuate slots at the ribs.

The handle assembly 402 includes a rear gripping member 450. It forms the opposing attachment location for the ribs 412 and 414, opposite the base ring 410. The gripping member can be any acceptable size that provides ergonomic support for a practitioner during a procedure. In one embodiment the member 450 has an outer diameter of approximately 12.7 to 19.05 mm (1/2 to 3/4 inch) and an external length of approximately 101.6 to 127 mm (4 to 5 inches). However, it is expressly contemplated that both these dimensions are widely variable outside the stated ranges herein. The member 450 defines an inner cylindrical barrel 452 having an inner diameter sized to slideably receive and guide the proximal end of the actuator handle 150. The barrel 452 has a sufficient length relative to the inner wall 462 of its end cap 460 so that the end 160 of the device does not strike the wall 462 at maximum withdrawal (as approximately shown) of the loop(s) 122 into the sheath 102.

Coatings can be applied to the outer surfaces of the core assembly and the sheath assembly to reduce friction between the core and the tube as well as to enhance movement of the snare device within a catheter. In one embodiment, a lubricious coating, such as PTFE (Teflon), hydrophilic, or diamond-like coating (DLC) may be applied to the outer surface of the sheath to reduce friction. Likewise, one of these coatings may be applied to the outer surface of the core wire to reduce friction with respect to the sheath. Since the coating adds a quantifiable thickness to the thickness of the sheath and/or diameter of the core wire, the overall size of components should be adjusted to compensate for the thickness of any lubricating coating. For example, the outer diameter of the sheath may need to be reduced to maintain a desired 889 µm (0.03 5-inch) or less outer diameter. Likewise, the thickness of the uncoated wall of the sheath may be reduced to maintain the desired inner diameter and create a final wall thickness, with coating, of approximately 50.8 µm (0.0020 inch).

According to an alternate embodiment, as shown in Fig. 6, the loop wire strand 602 (solid in this example) may be made from a half-round or "D-shaped" profile, at least in the vicinity of its joint with the core wire. Note that the advantages of this structure are particularly advantageous in the embodiment described above where the core wire distal end actually forms the loop strand and is joined back on itself so that a separate overlapping core wire end (joined to *two* separate loop ends) is not present. This D-shaped profile allows for maximizing the cross sectional area of the loop wire thereby increasing its overall breaking strength. For example, a tube with a 203.2 µm (0.008-inch) inner diameter can accommodate two 101.6 µm (0.004-inch) diameter round wires stacked together, or the equivalent of a single 203.2 µm (0.008-inch) (approximately diameter wire if two "D-shaped" wires are stacked. For a given overall desired diameter of DW, the wire strands are half-circular cross sections joined at a line, each having the individual width 1/2DW taken through a center point 606 and normal to the joint line between halves. The total cross sectional area of a 101.6 µm (0.004-inch) diameter wire is 8,107.3 µm² (0.000013 square inch), whereas the joined "D-shaped" wire has a cross sectional area 15,590 µm² (0.000025 square inch). This results in a doubling of the cross sectional area, and likewise, doubling of the breaking strength of the wire.

Having described the general structure of the snare device and it's various alternate embodiments, the operation of the snare device is now briefly described. In use, the loop 122 of the snare device is first withdrawn (proximally) into the sheath 102 by pulling on the actuating handle 150. The snare is then advanced into a balloon or guiding catheter (not shown) until the distal end 104 of the snare device has exited the distal end of the catheter. The snare device is then torqued and manipulated into place adjacent to an object to be retrieved. The snare loop 122 is then exposed (extended) from the open distal end 104 of the sheath 102 by pushing the actuating handle 150 forward (distally), and through a combination of advancing (distally), withdrawing (proximally), and rotating the entire device, the object is ensnared within the loop. The loop is then retracted/withdrawn back into the sheath so that the ensnared object is driven against the distal end 104 of the sheath and grasped tightly within the remaining exposed loop. With the object so-grasped, the snare device with the object is withdrawn (proximally) from the patient's body.

Having described the structure of a snare device according to various embodiments herein and some exemplary techniques for employing the device the following advantages, among others of the above-described invention should be clearer. Namely, this invention provides a small-diameter snare device, less than 889 µm (.035") diameter that is capable of fitting through existing balloon or guiding catheters. The body of the snare consists of a thin-walled polymer sheath, which allows for a maximized central core wire diameter, which in turn, provides stiffness for torque control and pushability in the body of the snare device. This device enables addition of one or more extensions onto the proximal end of the snare to allow for exchanging catheters directly over the snare if desired. Portions or all of the sheath and the snare loops can be radiopaque to aid in fluoroscopic visualization. Finally, lubricious coatings can be applied to the outer surface of the core wire and sheath to reduce friction and aid in movement.

The foregoing has been a detailed description of illustrative embodiments of the invention. Various modifications and additions can be made without departing from the spirit and scope of this invention. For example, while specified materials are described, it is expressly contemplated that similar or superior materials may be employed if and when available for the described components of this invention. Likewise, alternate techniques and materials can be employed for joining components. In addition further attachments can be provided to the device, with appropriate mounting hardware and locations to facilitate other, non-described procedures using the device. Accordingly, this description is meant to be taken only by way of example, and not to otherwise limit the scope of the invention, as defined by the claims.

## Claims

1. A small-diameter snare comprising:
- a thin-walled outer sheath (102) including a proximal end (170) and a distal end (104); and
- a core wire (110) having, at a distal end (104), a loop (122) adapted to retract into and extend from the distal end of the sheath (102) to ensnare an object, and at a proximal end (170) an opposing actuator handle (150) that extends from the proximal end (170) of the sheath (102), the core wire (110) being constructed and arranged so that applying axial movement to the handle (150) causes the loop (122) to retract into and extend from the sheath (102), **characterized in that** said outer sheath (102) is constructed from polymer and has an outer diameter between 254µm (.010 inch) and 533.4µm (.021 inch).

2. The small-diameter snare as set forth in claim 1 wherein each of the handle and the sheath define a maximum outer diameter, the maximum outer diameter being less than an inner diameter of a receiving catheter adapted to guide the sheath and the handle into the receiving catheter and into a body region.

3. The small-diameter snare as set forth in claim 2 wherein a portion of the proximal end of the core wire, proximally of the handle includes a connector constructed and arranged to receive a removable extension having an outer diameter less than the inner diameter of the receiving catheter.

4. The small-diameter snare as set forth in claim 1 wherein the loop comprises a D-shaped cross section strand that joins together at a joint with the core wire to define the loop thereby and that, at a location of the joint, the joined strand defines an approximately circular cross section.

5. The small-diameter snare as set forth in claim 1 wherein at least one of an outer surface of the outer sheath an inner surface of the outer sheath and an outer surface of the core wire are coated with a lubricating coating.

6. The small-diameter snare as set forth in claim 1 wherein the loop includes a radiopaque material thereon.

7. The small-diameter snare as set forth in claim 6 wherein the radiopaque material is located on a marker mounted on at least a portion of the loop.

8. The small-diameter snare as set forth in claim 1 wherein the loop defines a figure eight twisted structure having a plurality of loop members.

9. The small-diameter snare as set forth in claim 6 wherein the loop comprises a plurality of bundled strands and wherein at least one of the strands is radiopaque.

10. The small-diameter snare as set forth in claim 1 wherein the loop comprises a pair of proximal ends that are attached to the distal end of the core wire by a helical wire wrap that has, flowed past gaps in the helical wire wrap therethrough, a joining material.

11. The small-diameter snare as set forth in claim 10 wherein the joining material comprises at least one of solder, braze and weld.

12. The small-diameter snare as set forth in claim 1 wherein the sheath includes along at least a portion thereof a radiopaque coating.

13. The small-diameter snare as set forth in claim 1 wherein the polymer comprises polyimide material.

14. The small-diameter snare as set forth in claim 13 wherein the polyimide material includes a tungsten filler for providing radiopacity.

15. The small-diameter snare as set forth in claim 14 wherein the sheath includes on an outer surface thereon a PTFE coating.

## Patentansprüche

1. Schlinge mit kleinem Durchmesser, welche enthält:
eine dünnwandige Außenhülle (102), welche ein proximales Ende (170) und ein distales Ende (104) enthält; und
einen Kerndraht (110), welcher an einem distalen Ende (104) eine Schleife (122), welche dazu ausgelegt ist, sich in das distale Ende von der Hülle (102) einzuziehen und daraus zu erstrecken, um ein Objekt zu umgarnen, und an einem proximalen Ende (170) ein gegenüberliegendes Betätigungs-Griffstück (150) hat, welches sich von dem proximalen Ende (170) von der Hülle (102) erstreckt, wobei der Kerndraht (110) derart aufgebaut und angeordnet ist, so dass das Anlegen von einer axialen Bewegung auf das Griffstück (150) bewirkt, dass sich die Schleife (120) in die Hülle (102) einzieht und daraus erstreckt, **dadurch gekennzeichnet, dass**
die Außenhülle (102) aus Polymer erstellt ist und einen Außendurchmesser zwischen 254 µm (0,010 Inch) und 533,4 µm (0,021 Inch) hat.

2. Schlinge mit kleinem Durchmesser nach Anspruch 1, bei welcher das Griffstück und die Hülle einen maximalen Außendurchmesser bestimmen, wobei der maximale Außendurchmesser kleiner als ein Innendurchmesser von einem Aufnahme-Katheder ist, welcher dazu ausgelegt ist, die Hülle und das Griffstück in den Aufnahme-Katheder und in einen Körperbereich zu führen.

3. Schlinge mit kleinem Durchmesser nach Anspruch 2, bei welcher ein Abschnitt von dem proximalen Ende von dem Kerndraht nahe dem Griffstück einen Verbinder enthält, welcher dazu aufgebaut und angeordnet ist, um eine neu bewegbare Erweiterung aufzunehmen, welche einen Außendurchmesser hat, welcher kleiner als der Innendurchmesser von dem Aufnahme-Katheder ist.

4. Schlinge mit kleinem Durchmesser nach Anspruch 1, bei welcher die Schleife eine Litze enthält, welche im Querschnitt D-förmig geformt ist, welche an einem Verbindungspunkt mit dem Kerndraht zusammen verbunden ist, um **dadurch** die Schleife zu bestimmen, und wobei an einer Position von dem Verbindungspunkt die verbundene Litze einen ungefähr kreisförmigen Querschnitt bestimmt.

5. Schlinge mit kleinem Durchmesser nach Anspruch 1, bei welcher eine Außenoberfläche von der Außenhülle und/oder eine Innenoberfläche von der Außenhülle und eine Außenoberfläche von dem Kerndraht mit einer Schmierbeschichtung beschichtet sind.

6. Schlinge mit kleinem Durchmesser nach Anspruch 1, bei welcher die Schleife ein röntgendichtes Material darauf enthält.

7. Schlinge mit kleinem Durchmesser nach Anspruch 6, bei welcher das röntgendichte Material an einem Markierungsabschnitt positioniert ist, welcher auf zumindest einem Abschnitt von der Schleife befestigt ist.

8. Schlinge mit kleinem Durchmesser nach Anspruch 1, bei welcher die Schleife eine achtförmig verdrillte Struktur bestimmt, welche eine Mehrzahl von Schleifenelementen hat.

9. Schlinge mit kleinem Durchmesser nach Anspruch 6, bei welcher die Schleife eine Mehrzahl von gebündelten Litzen enthält, und bei welcher zumindest eine von den Litzen röntgendicht ist.

10. Schlinge mit kleinem Durchmesser nach Anspruch 1, bei welcher die Schleife ein Paar von proximalen Enden enthält, welche an dem distalen Ende von dem Kerndraht durch eine spiralförmige Drahthülle befestigt sind, welche, stromabwärts von Spalten in der spiralförmigen Drahthülle **dadurch**, ein Verbindungsmaterial hat.

11. Schlinge mit kleinem Durchmesser nach Anspruch 10, bei welcher das Verbindungsmaterial zumindest eines enthält aus Lötzinn, Lötmaterial und eine Schweißnaht.

12. Schlinge mit kleinem Durchmesser nach Anspruch 1, bei welcher die Hülle entlang von zumindest einem Abschnitt davon eine röntgendichte Beschichtung enthält.

13. Schlinge mit kleinem Durchmesser nach Anspruch 1, bei welcher das Polymer ein Polyimid-Material enthält.

14. Schlinge mit kleinem Durchmesser nach Anspruch 13, bei welcher das Polyimid-Material einen Wolfram-Füllstoff zum Bereitstellen von einer Röntgenstrahlenundurchlässigkeit enthält.

15. Schlinge mit kleinem Durchmesser nach Anspruch 14, bei welcher die Hülle an einer Außenoberfläche davon eine PTFE-Beschichtung enthält.

## Revendications

1. Anse de petit diamètre, comprenant :
- une gaine extérieure (102) à paroi mince incluant une extrémité proximale (170) et une extrémité distale (104) ; et
- un fil d'âme (110) ayant, à son extrémité distale (104), une boucle (122) adaptée à se rétracter dans et à se déployer hors de l'extrémité distale de la gaine (102) pour enserrer un objet, et à une extrémité proximale (70), une poignée d'actionnement opposée (150) qui s'étend depuis l'extrémité proximale (170) de la gaine (102), le fil d'âme (110) étant construit et agencé de telle façon que l'application d'un mouvement axial à la poignée (150) amène la boucle (122) à se rétracter à l'intérieur et à se déployer hors de la gaine (102), **caractérisée en ce que** ladite gaine extérieure (102) est réalisée en polymère et possède un diamètre extérieur entre 254 µm (0,010 pouce) et 533,4 µm (0,021 pouce).

2. Anse de petit diamètre selon la revendication 1, dans laquelle chacun des éléments que sont la poignée et la gaine définit un diamètre extérieur maximum, le diamètre extérieur maximum étant inférieur à un diamètre intérieur d'un cathéter de réception adapté à guider la gaine et la poignée à l'intérieur du cathéter de réception et jusque dans une région corporelle.

3. Anse de petit diamètre selon la revendication 2, dans laquelle une portion de l'extrémité proximale du fil d'âme, en situation proximale par rapport à la poignée, inclut un connecteur construit et agencé pour recevoir une extension amovible ayant un diamètre extérieur inférieur au diamètre intérieur du cathéter de réception.

4. Anse de petit diamètre selon la revendication 1, dans laquelle la boucle comprend un brin à section transversale en forme de D qui est réuni, au niveau d'une jonction, ensemble avec le fil d'âme pour définir la boucle à ce niveau et dans laquelle, à l'emplacement de la jonction, le brin réuni définit une section transversale approximativement circulaire.

5. Anse de petit diamètre selon la revendication 1, dans laquelle une surface au moins parmi la surface extérieure de la gaine extérieure, la surface intérieure de la gaine extérieure, et une surface extérieure du fil d'âme est revêtue avec un revêtement lubrifiant.

6. Anse de petit diamètre selon la revendication 1, dans laquelle la boucle inclut sur elle-même un matériau radioopaque.

7. Anse de petit diamètre selon la revendication 6, dans laquelle le matériau radioopaque est situé sur un marqueur monté sur au moins une portion de la boucle.

8. Anse de petit diamètre selon la revendication 1, dans laquelle la boucle définit une structure tordue en forme de chiffre huit ayant une pluralité d'éléments de boucle.

9. Anse de petit diamètre selon la revendication 6, dans laquelle la boucle comprend une pluralité de brins en faisceau, et dans laquelle l'un au moins des brins est radioopaque.

10. Anse de petit diamètre selon la revendication 1, dans laquelle la boucle comprend une paire d'extrémités proximales qui sont attachées à l'extrémité distale du fil d'âme par un fil bobiné hélicoïdal qui comprend un matériau de jonction, lequel s'écoule en traversant des interstices dans le fil bobiné hélicoïdal.

11. Anse de petit diamètre selon la revendication 10, dans laquelle le matériau de jonction comprend au moins un matériau parmi matériau de soudure, de brasage et de soudure autogène.

12. Anse de petit diamètre selon la revendication 1, dans laquelle la gaine inclut un revêtement radioopaque le long d'au moins une portion d'elle-même.

13. Anse de petit diamètre selon la revendication 1, dans laquelle le polymère comprend un matériau polyimide.

14. Anse de petit diamètre selon la revendication 13, dans laquelle le matériau polyimide inclut une charge de tungstène pour assurer la radioopacité.

15. Anse de petit diamètre selon la revendication 14, dans laquelle la gaine inclut un revêtement de polytétrafluoroéthylène sur une surface extérieure d'elle-même.
